# EUROPÄISCHE PATENTSCHRIFT

(11) **EP 3 217 939 B1**
(45) Veröffentlichungstag und Bekanntmachung des Hinweises auf die Patenterteilung: **21.07.2021**
(21) Anmeldenummer: 15793711.1
(22) Anmeldetag: 10.11.2015
(51) Int. Cl.: A61G 12/00, F16M 11/04, G02B 7/00, F16M 11/08, A61B 90/50, F16M 13/02

(54) **ABSTÜTZGELENK FÜR EINEN TRAGARM EINER MEDIZINTECHNISCHEN STATIVVORRICHTUNG**
SUPPORT JOINT FOR A CANTILEVER OF A MEDICAL STAND DEVICE
ARTICULATION D'APPUI POUR UN ARBRE DE SUPPORT D'UN DISPOSITIF À PIED MÉDICAL

(30) Priorität: 10.11.2014 EP 14003775
(43) Veröffentlichungstag der Anmeldung: 20.09.2017
(73) Patentinhaber: Ondal Medical Systems GmbH, 36088 Hünfeld (DE)
(72) Erfinder: OGINSKI, Stefan, 36041 Fulda (DE); BARTON, Joachim, 36039 Fulda (DE); EULER, Annika, 70499 Stuttgart (DE)
(74) Vertreter: Graf von Stosch, Andreas
(86) Internationale Anmeldenummer: PCT/EP2015/002255
(87) Internationale Veröffentlichungsnummer: WO 2016/074787

(56) Entgegenhaltungen:
- DE-B3- 10 314 156
- DE-U1- 20 018 361
- DE-U1-202011 005 203
- US-A1- 2007 080 275
- US-A1- 2011 315 843
- US-A1- 2012 235 000

## Beschreibung

Die vorliegende Erfindung betrifft ein Tragsystem zum Halten und Abstützen einer medizintechnischen Einrichtung mit vordefinierter Masse, wobei eine Nutzlast der Tragarmgelenkvorrichtung in Abhängigkeit der von der medizintechnischen Einrichtung ausgeübten Gewichtskraft einstellbar ist. Je nach Hebelarm übt die Masse dabei auch ein Moment auf die Tragarmgelenkvorrichtung aus. Die vorliegende Erfindung betrifft ein Tragsystem bzw. eine Stativvorrichtung jeweils mit einzelnen Merkmalen des entsprechenden unabhängigen Anspruchs, sowie die Verwendung eines solchen Tragsystems an einer medizintechnischen Stativvorrichtung, insbesondere im Operationssaal.

Stative, insbesondere Deckenstative wie z.B. Deckenversorgungseinheiten, Monitorträger, oder so genannte Federarme oder Zentralachsen, weisen meist einen oder mehrere in Bezug auf eine Vertikalposition starr angeordneten oder höhenverstellbaren Träger auf, mittels welchen eine daran befestigte medizintechnische Einrichtung bewegt und positioniert werden kann, z.B. im Operationssaal, insbesondere auch auf einer Intensivstation. An den Stativen sind häufig Versorgungseinheiten montiert, an welchen z.B. medizinisch-elektrische Endgeräte angeordnet sind, die z.B. während einer Operation mit den benötigten Medien versorgt werden. Bei den Endgeräten kann es sich auch um einen (Diagnose-)Monitor oder eine Beleuchtungseinrichtung handeln. Die Träger oder Versorgungseinheiten sind meist drehbar und/oder höhenverstellbar und/oder um eine zumindest annähernd horizontal ausgerichtete Achse in der Höhe verschwenkbar gelagert. Die Träger können je nach Funktion oder Ausgestaltung auch als Ausleger, Tragarm oder Federarm bezeichnet werden.

Bei solchen Stativen ist es erforderlich, die medizintechnischen Einrichtungen bzw. Endgeräte schwebend zu halten, wobei in einem jeweiligen Träger eine Gegenkraft aufgebracht werden muss, welche der Gewichtskraft des Endgerätes entgegenwirkt. Je nach Masse des Endgerätes ist das Einstellen der Gegenkraft erforderlich. Das Einstellen sollte dabei in Hinblick auf einen möglichst großen Bereich von unterschiedlichen Gewichtskräften bzw. Massen erfolgen können, und zwar ohne die Bewegungsfreiheit des Trägers/Tragarms zu beeinträchtigen.

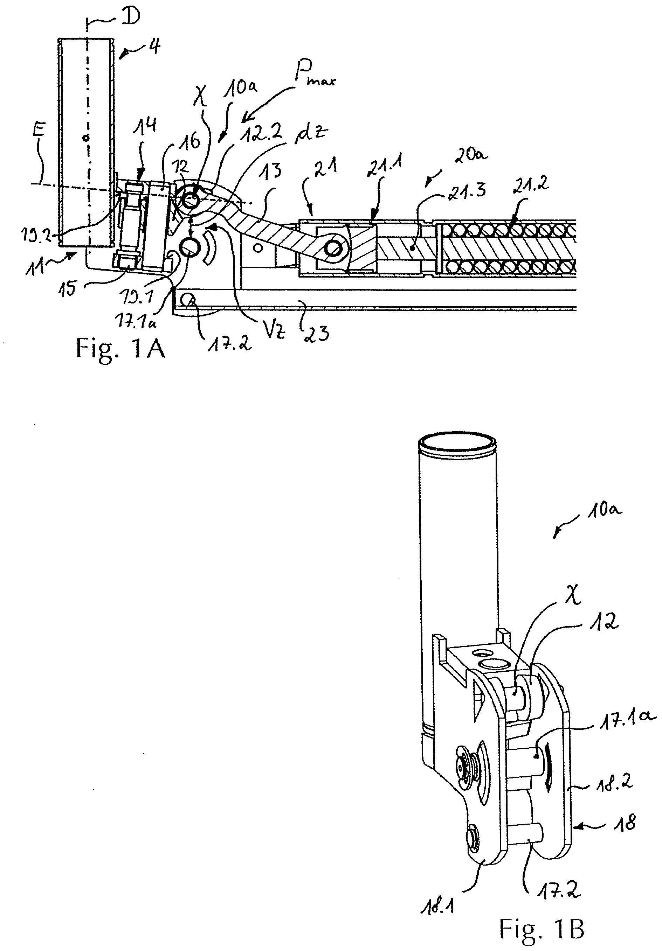

Zum Ausbalancieren der medizintechnischen Einrichtung und zum Entgegenwirken auf deren Masse kann beispielsweise eine druck- oder zugfederunterstützte Kinematik verwendet werden, die in Form eines Parallelogramms ausgebildet ist. Der Tragarm weist dann zwei Streben auf, welche parallelogrammartig angeordnet sind und mit ihren Enden jeweils in zwei Schwenkachsen gelagert sind. Die Streben erstrecken sich dann zwischen zwei Tragarmgelenken, welche jeweils zwei übereinander angeordnete Schwenkachsen definieren. Die Feder ist dabei an einer der Streben angeordnet und dient als Energiespeicher und als krafterzeugende Komponente an der Strebe, um die aus der Masse/Gewichtskraft der medizintechnischen Einrichtung resultierenden Kraftkomponenten bzw. Momente in der Kinematik aufzunehmen. Die Kinematik ist so ausgelegt, dass der Federarm eingerichtet ist, die medizintechnische Einrichtung in der jeweiligen Höhenposition zu halten bzw. auszubalancieren, ohne eine zusätzliche Einstellung am Federarm vornehmen zu müssen. Der Tragarm kann aus der horizontalen Position manuell um die (jeweilige) Schwenkachse nach oben bzw. unten verschwenkt und an beliebiger Position angehalten werden, z.B. in einem Verschwenkbereich von +45° (nach oben) und -50° (nach unten). Diese Kinematik kann mittels einer in einem, insbesondere im hinteren Tragarmgelenk integrierten Einstellmechanik justiert werden. Eine solche Einstellmechanik ist im Detail in den Figuren 1A, 1B beschrieben.

DE 20 2011 005203 U1 beschreibt eine räumlich verstellbare Konsole, insbesondere ein Deckenstativ, welche zur Befestigung und zum Tragen von Aufhängezubehör, wie ärztlichen Instrumenten, dient.

DE 103 14 156 B3 beschreibt eine Schwenkhalterungsanordnung, welche ein erstes Kraftgerät aufweist, um ein Gegendrehmoment derart zu erzeugen, dass ein Schwenkarm in Position verharrt. Die Kraft des ersten Kraftgeräts wird durch ein ansteuerbares zweites Kraftgerät ergänzt.

US 2011/315843 A1 beschreibt eine Tragarmanordnung für einen Bildschirm, bei welchem der Bildschirm schnell von einem Tragarm entkoppelt werden kann.

US 2007/080275 A1 beschreibt eine Vorrichtung zur Stabilisierung von medizinischen Geräten, wenn diese manuell verlagert werden.

DE 200 18 361 U1 beschreibt eine Haltevorrichtung für einen Flüssigkristall-Bildschirm.

Es ist eine Aufgabe der vorliegenden Erfindung, ein Tragsystem für ein medizintechnisches Stativ bereitzustellen, womit der Einsatzbereich verbreitert und ein breites Spektrum an Nutzlasten eingestellt werden kann. Insbesondere besteht die Aufgabe darin, werksseitig wenigstens eine Komponente eines Tragsystems voreingestellt bereitzustellen, mittels welcher nicht nur besonders große Nutzlasten, sondern auch sehr kleine Nutzlasten eingestellt werden können, ohne dass konstruktive Änderungen an der Komponente vorgenommen werden müssen. Die Aufgabe kann auch darin gesehen werden, bei einem parallelogrammartigen Tragarm oder Federarm eine Verbindung zwischen einem Tragarm und einem (Schwenk-)Gelenk derart auszugestalten, dass eine Strebe des Tragarms an unterschiedlichen Positionen in einem möglichst großen Verstellbereich auf stabile Weise abgestützt werden kann, je nach gewünschter Nutzlast.

Diese Aufgabe wird durch ein Tragsystem mit den Merkmalen des Anspruchs 1, eine Stativvorrichtung mit den Merkmalen des Anspruchs 12 und eine Verwendung eines Tragsystems gemäß den Merkmalen des Anspruchs 13 gelöst.

Das Tragsystem dient für eine Stativvorrichtung zur Anordnung im Operationssaal und zum örtlichen Verlagern einer medizintechnischen Einrichtung mit vordefinierter Masse. Das Tragsystem umfasst wenigstens einen Tragarm zum Halten der medizintechnischen Einrichtung mit wenigstens einer jeweils in einer Schwenkachse gelagerten Strebe, wobei die Strebe mittels eines Hebels zusätzlich an einer Stützachse abgestützt ist. Zudem umfasst das Tragsystem eine Tragarmgelenkvorrichtung für den Tragarm der Stativvorrichtung zur Anordnung im Operationssaal und zum örtlichen Verlagern einer am Tragarm gehaltenen medizintechnischen Einrichtung, wobei die Tragarmgelenkvorrichtung zum Einstellen einer Nutzlast entsprechend einer vom Tragarm aufzunehmenden Gewichtskraft der medizintechnischen Einrichtung eingerichtet ist, umfassend: wenigstens eine Schwenkachse jeweils zum Lagern wenigstens einer Strebe des Tragarms; und eine Stützachse zum Abstützen eines Hebels eingerichtet zum Übertragen von Kräften, insbesondere von durch eine (Zug- oder Druck-)Feder hervorgerufenen Zugkräften, zwischen der Strebe und der Tragarmgelenkvorrichtung; wobei ein Abstand zwischen den Achsen (Achsabstand) zum Einstellen der Nutzlast in einem Verstellbereich justierbar ist; und wobei die Größe und/oder Erstreckung des Verstellbereichs unabhängig von der Position der Schwenkachse ist. Hierdurch kann ein großer Verstellbereich bzw. ein breites Spektrum von Nutzlasten ermöglicht werden.

Der vergleichsweise große Verstellbereich ermöglicht die Verwendung nur eines Federtyps als Energiespeicher für unterschiedliche Stative bzw. Hebelarmlängen bzw. Massen bzw. Nutzlastbereichen. Dabei kann werksseitig eine einheitliche Voreinstellung der Federkraft für mehrere Produkttypen realisiert werden. Eine Staffelung der Federkrafteinstellung in Hinblick auf spezifische Nutzlastbereiche ist damit nicht mehr erforderlich. Ein Anwender kann individuell die erforderliche Nutzlast des Federarms über den gesamten Nutzlastbereich am Einsatzort einstellen. Dies ermöglicht nicht zuletzt, bei einem für vergleichsweise große Nutzlasten voreingestellten Tragarm den Tragarm auch auf vergleichsweise kleine Nutzlasten nachzujustieren/einzustellen.

Der Abstand zwischen den Achsen (Achsabstand) definiert dabei den Hebelarm, mit welchem eine von der Kinematik bzw. einer Feder des Tragarms ausgeübte Kraft am Tragarmgelenk angreift. Der Abstand zwischen den Achsen kann dabei auch den relativen Winkel des Hebels definieren. Die vorliegende Erfindung beruht auf dem Konzept, die Tragarmgelenkvorrichtung des erfindungsgemäßen Tragsystems derart auszugestalten, dass der Achsabstand minimierbar ist, insbesondere gegen NULL konvergieren kann. Hierzu wird bevorzugt ein besonders großer Verstellbereich bereitgestellt.

Mit anderen Worten: Die erfindungsgemäß im Tragsystem eingesetzte Tragarmgelenkvorrichtung kann auch eine unternehmensinterne Verringerung von Produktionsvarianten ermöglichen. Eine Anwendungsplanung kann vereinfacht werden, denn Tragarme/Federarme des gleichen Typs müssen nicht mehr nach Nutzlastkategorien klassifiziert werden. Auch kann ein Risiko einer Verwechslung von Federarmen des gleichen Typs mit spezifischen Nutzlastbereichen vermindert werden.

Die erfindungsgemäß im Tragsystem eingesetzte Tragarmgelenkvorrichtung kann eingerichtet sein, die einstellbare/justierbare Nutzlast zu minimieren, indem der Verstellbereich maximiert ist. Die erfindungsgemäß eingesetzte Tragarmgelenkvorrichtung kann eingerichtet sein, die einstellbare/justierbare Nutzlast zu minimieren, indem der Achsabstand minimierbar ist.

Als Stativvorrichtung gemäß Anspruch 12 ist dabei eine Vorrichtung zum Halten, ortsfesten Anordnen und/oder Verlagern mindestens einer medizintechnische Einrichtung zu verstehen, die bevorzugt an einer Wand (in einem Wandlager) oder einer Raumdecke oder auch am Boden eines Operationssaals oder irgendeines anderen Raumes für medizinische Zwecke fest montiert oder positioniert werden kann, also z.B. ein Deckenstativ. Die Stativvorrichtung ist dann nicht vollkommen frei im Operationssaal verlagerbar, sondern kann nur in einem bestimmten Aktionsradius verlagert werden, insbesondere relativ zu einem an einer Raumdecke oder Wand des Operationssaals angeordneten Befestigungspunkt bzw. Montagepunkt. Die Stativvorrichtung kann als an einer Raumdecke montierte Deckenversorgungseinheit ausgebildet sein und eine oder mehrere Versorgungskonsolen aufweisen, welche an einem oder zwei Tragarmen gelagert und positionierbar ist. Die Stativvorrichtung kann auch als Monitorträger ausgebildet sein. Die Stativvorrichtung kann auch als so genannter, insbesondere an einer Wand montierter Federarm ausgebildet sein und z.B. eine Leuchte aufweisen. Ein Federarm weist dabei eine bewegbare, insbesondere höhenverstellbare Mechanik auf, die eine Last in verschiedenen Höhen halten kann und unter dem Einsatz geringer Bedienkräfte (bis ca. 50 N) manuell verstellbar ist. Die Stativvorrichtung kann auch als so genannte, insbesondere an einer Raumdecke montierte Zentralachse ausgebildet sein und eine Mehrzahl von Tragsystemen mit jeweils mindestens einem Träger aufweisen, an welchem z.B. ein Monitor oder eine Leuchte gelagert ist. Bevorzugt weist die Stativvorrichtung mindestens zwei Tragarme auf.

Als medizintechnische Einrichtung ist dabei bevorzugt eine Versorgungskonsole zu verstehen, mittels welcher Mittel für eine Versorgung eines Patienten und/oder Instrumente für einen Operateur und/oder Licht, Reinluft oder andere im Operationssaal benötigte Medien bereitgestellt werden können. Die medizintechnische Einrichtung weist bevorzugt irgendein Bedienpanel und/oder irgendeine Anzeigevorrichtung zum grafischen Darstellen von z.B. Patientendaten auf.

Als Operationssaal ist dabei auch ein Untersuchungsraum oder eine Intensivstation aufzufassen, also ein Raum zum Durchführen medizinischer Behandlungen oder Therapien.

Als Schwenkachse ist dabei bevorzugt eine Achse zu verstehen, um welche eine Strebe eines Tragarms in der Höhe nach oben oder unten verschwenkt werden kann. Die Schwenkachse erstreckt sich bevorzugt in einer horizontalen Ebene, insbesondere orthogonal zu einer Drehachse, um welche herum der Tragarm/Federarm bzw. die Tragarmgelenkvorrichtung in horizontaler Ebene gelagert sein kann.

Als Stützachse ist dabei bevorzugt eine Achse zu verstehen, welche eingerichtet ist, (Druck- oder Zug-)Kräfte vom Tragarm auf das Tragarmgelenk zu übertragen und in ein Gehäuse des Tragarmgelenks weiterzuleiten. Die Stützachse kann verlagerbar sein und ist selbst am Tragarmgelenk abgestützt.

Gemäß einem Ausführungsbeispiel ist die Schwenkachse derart relativ zur Stützachse angeordnet und/oder dimensioniert, dass Schwenk- und Stützachse nicht überlappen, insbesondere in Bezug auf deren Achsrichtung. Schwenk- und Stützachse können in Achsrichtung relativ zueinander vollständig versetzt angeordnet sein. Hierdurch kann ein Verstellbereich bereitgestellt werden, welcher sich über einen maximal großen Bereich der Gelenkvorrichtung erstreckt. Die Schwenkachse kann seitlich vom Verstellbereich angeordnet sein. Gemäß einer Variante sind Schwenk- und Stützachse parallel zueinander ausgerichtet.

Gemäß der Erfindung ist die Schwenkachse in Abschnitte unterteilt, so dass die relative Position der Stützachse relativ zur Schwenkachse unabhängig von der Position der Schwenkachse oder des Hebels einstellbar ist, insbesondere der Abstand minimierbar ist. Zusätzlich kann die Schwenkachse derart dimensioniert sein und/oder in Abschnitte geteilt sein, dass die relative Position der Stützachse relativ zur Schwenkachse unabhängig von der Position der Schwenkachse oder des Hebels einstellbar ist, insbesondere der Abstand minimierbar ist. Hierdurch kann eine symmetrische Abstützung erfolgen, insbesondere an zwei möglichst weit voneinander beabstandeten, außenliegenden Achsabschnitten. Der Abstand kann unabhängig von der Anordnung der Achsen minimiert werden, so dass eine besonders kleine Nutzlast einstellbar ist.

Als Achsenabschnitt ist dabei bevorzugt ein Teil einer Achse zu verstehen, welcher separat von einem anderen Teil derselben Achse angeordnet und abgestützt ist. Die Achse kann dabei in zwei oder mehrere Abschnitte unterteilt sein. Jeder Abschnitt kann dabei dieselbe Funktion erfüllen und ist bevorzugt in derselben Richtung ausgerichtet.

Gemäß der Erfindung ist die Schwenkachse in wenigstens zwei Abschnitte geteilt, zwischen welchen eine Kavität bzw. ein Freiraum gebildet ist, insbesondere zentrisch in der Tragarmgelenkvorrichtung, wobei der Verstellbereich in der Kavität angeordnet ist, wobei die Kavität bevorzugt zur teilweisen Aufnahme des Hebels zwischen den Achsenabschnitten oder neben einen jeweiligen Achsenabschnitt eingerichtet ist. Hierdurch kann ein vergleichsweise robustes, großes Lagerelement auch derart angeordnet werden, dass eine symmetrische Einleitung von Abstützkräften erfolgen kann.

Dabei kann die Stützachse derart kurz sein, dass die Stützachse neben der Schwenkachse oder in einer/der Kavität zwischen einzelnen Abschnitten der Schwenkachse positionierbar ist, insbesondere in einem Gehäuse der Tragarmgelenkvorrichtung. Hierdurch kann die Anordnung und Abstützung der Stützachse optimiert werden, insbesondere in einem Gussgehäuse.

Gemäß einem Ausführungsbeispiel ist der Verstellbereich durch ein Gehäuse der Tragarmgelenkvorrichtung eingegrenzt, insbesondere nach oben und nach unten. Die Länge der Schwenkachse ist bevorzugt kleiner als die Breite des Gehäuses. Hierdurch kann die Stützachse selbst auch im Gehäuse abgestützt werden. Dies ermöglicht eine besonders robuste Anordnung.

Gemäß einem Ausführungsbeispiel umfasst die im Tragsystem eingesetzte Tragarmgelenkvorrichtung ferner ein Lagerelement/Abstützelement, insbesondere einen Lagerbock, welches die Position der Stützachse definiert und an den Hebel kuppelbar/gekuppelt ist, insbesondere mittels der Stützachse; sowie Justagemittel zum Einstellen/Justieren des Abstands zwischen den Achsen im Verstellbereich, wobei das Lagerelement an die Justagemittel gekuppelt ist, insbesondere formschlüssig. Hierdurch kann die Stützachse auf exakte Weise in unterschiedlichen Positionen im Verstellbereich angeordnet und unabhängig von der jeweiligen Position auf stabile Weise abgestützt werden. Dabei kann der Achsabstand bevorzugt unabhängig von der geometrischen Ausgestaltung oder Dimension des Lagerelements minimiert werden, so dass eine besonders kleine Nutzlast einstellbar ist.

Als Lagerelement ist dabei bevorzugt ein Teil zu verstehen, mittels welchem eine Achse abgestützt und verschiebbar in unterschiedlichen Positionen gelagert werden kann. Das Lagerelement kann die mechanische Schnittstelle zwischen der Achse und einer in einer festen Position am Tragarmgelenk angeordneten Komponente sicherstellen.

Als Justagemittel ist dabei bevorzugt ein manuell oder maschinell einstellbarer Mechanismus zu verstehen, mittels welchem eine relative Position einer der Achse im Tragarmgelenk justierbar/einstellbar ist. Die Justagemittel können das Lagerelement bevorzugt formschlüssig positionieren, zumindest im Wesentlichen formschlüssig. Eine mechanische Schnittstelle zwischen den Justagemitteln und dem Lagerelement basiert bevorzugt im Wesentlichen auf Formschluss. Die Justagemittel können in sehr einfacher oder robuster Ausgestaltung als Stellschraube ausgeführt sein. Wahlweise kann z.B. auch ein Rastmechanismus oder eine Schiene mit unterschiedlichen diskreten Relativpositionen zum Einsatz kommen.

Gemäß einem Ausführungsbeispiel sind die Justagemittel eingerichtet, das Lagerelement derart an der Schwenkachse vorbei zu führen und/oder derart in einer/der Kavität neben der Schwenkachse zu positionieren, dass der Achsabstand minimiert ist. Hierdurch kann eine vergleichsweise kleine Nutzlast eingestellt werden, selbst bei einem vergleichsweise großen, werksseitig voreingestellten Maximalwert der Nutzlast. Es kann eine exakte Positionierung der Stützachse im gesamten Verstellbereich erfolgen, insbesondere auch direkt oberhalb von der Schwenkachse bei minimalen Hebelkräften. Dabei erstrecken sich die Justagemittel bevorzugt in einer Richtung orthogonal zur Schwenkachse an der Schwenkachse vorbei. Dies liefert eine vorteilhafte Kinematik, unabhängig vom Achsabstand.

Gemäß einem Ausführungsbeispiel definiert die im Tragsystem eingesetzte Tragarmgelenkvorrichtung, insbesondere ein Gehäuse der Tragarmgelenkvorrichtung, einen Anschlag, an welchem das Lagerelement in einer Anordnung mit minimalem Abstand zwischen den Achsen zur Anlage kommen kann, wobei der Anschlag bevorzugt unterhalb von der Schwenkachse angeordnet ist. Das Lagerelement kann/soll dabei nicht an der Schwenkachse zur Anlage kommen. Es besteht somit kein Risiko, dass das Lagerelement die Schwenkachse blockiert. Hierdurch kann auch ein vergleichsweise großer, stabiler Lagerbock verwendet werden.

Gemäß einem Ausführungsbeispiel erstreckt sich der Verstellbereich zwischen einer obersten Lagerelement-Position und einer untersten Lagerelement-Position bis unterhalb von der (oberen) Schwenkachse. Der Verstellbereich bzw. eine entsprechende Kavität zur Aufnahme eines Lagerelements kann sich an der Schwenkachse vorbei erstrecken, so dass der Achsabstand minimiert werden kann.

Gemäß einem Ausführungsbeispiel ist wenigstens ein separater Abschnitt der Schwenkachse separat in einer Gehäusewandung und bevorzugt auch in einer korrespondierenden Gehäuselasche gelagert, welche jeweils an einer Seite vom Verstellbereich angeordnet ist. Wenigstens ein Abschnitt der Schwenkachse kann in einer weiteren Gehäusewandung bzw. weiteren Gehäuselasche der Tragarmgelenkvorrichtung gelagert sein, wobei die Gehäusewandungen bevorzugt eine Außenkontur der Tragarmgelenkvorrichtung definieren. Die Strebe kann dann mit jeweiligen Laschen oder Fortsätzen zwischen der Gehäusewandung und der Gehäuselasche gelagert und geführt sein, wodurch eine hohe Stabilität sichergestellt werden kann.

Gemäß einem Ausführungsbeispiel umfasst die im Tragsystem eingesetzte Tragarmgelenkvorrichtung ferner Führungsmittel eingerichtet zum Führen des Lagerelements entlang einer vordefinierten Bewegungsbahn im Verstellbereich, insbesondere entlang einer Geraden. Das Lagerelement kann mittels der Führungsmittel in Position gehalten werden und/oder entlang einer z.B. vertikal ausgerichteten Positionslinie geführt werden. Das Lagerelement kann sowohl mit den Führungsmitteln als auch mit den Justagemitteln gekuppelt sein. Führungsmittel und Justagemittel können sich in derselben Richtung erstrecken, insbesondere parallel zueinander.

Als Führungsmittel ist dabei bevorzugt ein ausreichend dimensioniertes und abgestütztes Element bzw. Teil zu verstehen, welches in der Tragarmgelenkvorrichtung gelagert und abgestützt ist, und mittels welchem Stützkräfte von einer Strebe auf die Gelenkvorrichtung in unterschiedlichen Relativpositionen einer Stützachse übertragen werden können. Das Führungsmittel kann dabei auch ein Lager für die Stützachse bzw. für ein Abstützelement bereitstellen, insbesondere ein Gleitlager. Die Führungsmittel können z.B. in der Art einer Schlittenführung oder einer Säulenführung ausgestaltet sein. Die Führungsmittel können dabei zumindest in einer Kraftrichtung, insbesondere in einer Horizontalebene, auch vollständig eine Abstützung des Lagerelements übernehmen und dann auch als Abstützmittel bezeichnet werden.

Gemäß einem Ausführungsbeispiel sind die Justagemittel zwischen den Führungsmitteln und der Schwenkachse angeordnet. Dies ermöglicht eine vorteilhafte Spannungsverteilung im Gelenkkörper bzw. Gehäuse. Die Nutzlast kann dabei auf vergleichsweise direkte Weise eingestellt werden, denn die Justagemittel können dabei in unmittelbarer Nähe zur Stützachse angeordnet werden. Ferner kann verhindert werden, dass vertikale Kraftkomponenten auf die Führungsmittel übertragen werden. Bei der im Stand der Technik realisierten Anordnung kann aufgrund einer Verklemmung des Lagerbocks an den Führungsmitteln nicht ausgeschlossen werden, dass auch vertikale Kraftkomponenten auf die Führungsmittel übertragen werden. Dieser Effekt kann jedoch durch die Vertauschung der Positionen verringert oder vollständig eliminiert werden. Mit anderen Worten: Führungsmittel, welche vom Hebel bzw. vom Federarm aus gesehen hinter den Justagemitteln angeordnet sind, brauchen im Wesentlichen nur in Hinblick auf Horizontalkraftkomponenten ausgelegt werden. Eine Abstützung in vertikaler Richtung ist nicht notwendigerweise erforderlich. Vertikalkräfte können bereits durch die Justagemittel aufgenommen werden.

Gemäß einem Ausführungsbeispiel sind die Justagemittel und/oder die Führungsmittel in montierter Anordnung der Tragarmgelenkvorrichtung vertikal ausgerichtet/ausrichtbar, insbesondere parallel zu einer Drehachse der Tragarmgelenkvorrichtung. Dies ermöglicht, die Arm-Kinematik auf vergleichsweise einfache Weise an den großen Verstellbereich anzupassen und kann eine (Nach-)Bearbeitung der Gelenkvorrichtung erleichtern.

Gemäß einem Ausführungsbeispiel ist die im Tragsystem eingesetzte Tragarmgelenkvorrichtung ein Gussteil, insbesondere aus Aluminium. Hierdurch kann die Tragarmgelenkvorrichtung funktionsoptimiert, robust und materialsparend ausgestaltet werden. Insbesondere kann ein robustes Gehäuse bereitgestellt werden in welchem die (obere) Schwenkachse bzw. einzelne Achsabschnitte gelagert und abgestützt werden können. Mit dem großen Verstellbereich geht auch ein vergrößerter Bereich einher, in welchem die Kräfte von der Stützachse auf das Gehäuse übertragen werden, insbesondere mittels des Lagerbocks und eines/des Führungsbolzens. Ein Guss-Gehäuse kann die hierfür ausreichend steife Struktur bereitstellen. Eine Schweißkonstruktion hingegen müsste eine Vielzahl von Schweißnähten und Verstärkungen aufweisen und wäre nur als ein vergleichsweise aufwändiges Bauteil realisierbar.

Aluminium ist als Material bevorzugt. Wahlweise kann auch z.B. Zink oder Stahl oder Messing verwendet werden, insbesondere für ein Zinkdruckgussteil bzw. ein Stahlgussteil bzw. ein Messingdruckgussteil. Die Materialwahl kann dabei z.B. in Hinblick auf Festigkeit, Robustheit, Traglast oder Kosten erfolgen, je nach Anforderung.

Vorteilhaft, weist das Gehäuse der Tragarmgelenkvorrichtung, einen, dem Anschlag, an welchem das Lagerelement mit minimalem Abstand zwischen den Achsen zur Anlage kommen kann, gegenüberliegenden zweiten Anschlag auf, der eine Anschlagebene definiert. Das Lagerelement weist dabei einen Lagerfortsatz auf, der in einer der wenigstens einen Schwenkachse abgewandten Richtung von einem zentralen Teil des Lagerelements abragt, so dass die Stützachse in ihrer maximal von der wenigstens einen Schwenkachse beabstandeten Position im Bereich der Anschlagebene liegt. Die Anschlagebene ist dabei in einer durch den Verstellbereich vorgegebenen Richtung maximal entfernt von der wenigstens einen Schwenkachse.

In einem Ausführungsbeispiel ist es vorgesehen, dass die Tragarmgelenkvorrichtung für einen Tragarm einer Stativvorrichtung zur Anordnung im Operationssaal und zum örtlichen Verlagern einer am Tragarm gehaltenen medizintechnischen Einrichtung, wobei die Tragarmgelenkvorrichtung zum Einstellen einer Nutzlast entsprechend einer vom Tragarm aufzunehmenden Gewichtskraft der medizintechnischen Einrichtung eingerichtet ist, umfasst: wenigstens eine Schwenkachse jeweils zum Lagern wenigstens einer Strebe des Tragarms; eine Stützachse zum Abstützen eines Hebels eingerichtet zum Übertragen von den Tragarm haltenden Kräften zwischen der Strebe und der Tragarmgelenkvorrichtung; wobei ein Abstand zwischen den Achsen zum Einstellen der Nutzlast in einem Verstellbereich justierbar ist; wobei die Größe und/oder Erstreckung des Verstellbereichs unabhängig von der Position der Schwenkachse ist, und wobei die Schwenkachse derart relativ zur Stützachse angeordnet und/oder dimensioniert ist, dass Schwenk- und Stützachse nicht überlappen, insbesondere in Bezug auf deren Achsrichtung, und wobei die Schwenkachse in wenigstens zwei Abschnitte geteilt ist, neben oder zwischen welchen eine Kavität bzw. ein Freiraum/Hohlvolumen gebildet ist, insbesondere zentrisch in der Tragarmgelenkvorrichtung, wobei der Verstellbereich in der Kavität angeordnet ist; ferner umfassend: ein Lagerelement/Abstützelement, insbesondere Lagerbock, welches an der Stützachse gelagert ist und mit dem Hebel kuppelbar/gekuppelt ist; und Justagemittel zum Einstellen des Abstands zwischen den Achsen im Verstellbereich, wobei das Lagerelement an die Justagemittel gekuppelt ist, wobei die Kavität bevorzugt zur teilweisen Aufnahme des Hebels zwischen die Achsenabschnitte oder neben einen jeweiligen Achsenabschnitt eingerichtet ist, und wobei die Tragarmgelenkvorrichtung, insbesondere ein Gehäuse der Tragarmgelenkvorrichtung, einen Anschlag definiert, an welchem das Lagerelement in einer Anordnung mit minimalem Abstand zwischen den Achsen zur Anlage kommen kann, wobei der Anschlag bevorzugt unterhalb von der Schwenkachse angeordnet ist. Hierdurch ergeben sich bereits zuvor genannte Vorteile.

Gemäß der Erfindung weist das Tragsystem für die Stativvorrichtung zur Anordnung im Operationssaal und zum örtlichen Verlagern einer medizintechnischen Einrichtung mit vordefinierter Masse, umfassend wenigstens den Tragarm zum Halten der medizintechnischen Einrichtung mit wenigstens der jeweils in einer der Schwenkachsen gelagerten Strebe, wobei eine/die Strebe mittels des Hebels zusätzlich in einer Stützachse abgestützt ist, einen parallelogrammartigen Tragarm mit zwei übereinander in Richtung einer Längsachse des Tragarms bevorzugt parallel zueinander angeordneten Streben auf; sowie wenigstens die Tragarmgelenkvorrichtung mit wenigstens einer Schwenkachse und mit der Stützachse, wobei die Stützachse relativ zur Schwenkachse in einem Verstellbereich zum Einstellen einer Nutzlast der Tragarmgelenkvorrichtung bezüglich der zu haltenden Masse verlagerbar und positionierbar ist; wobei eine/die Strebe des Tragarms an wenigstens einem Abschnitt an der Schwenkachse neben dem Verstellbereich bzw. neben einer von der Tragarmgelenkvorrichtung definierten Kavität gelagert ist, in welchem/welcher die Stützachse angeordnet ist und in unterschiedlichen Positionen im Verstellbereich anordenbar ist, wobei die Schwenkachse räumlich vom Verstellbereich entkoppelt ist. Hierdurch ergeben sich bereits zuvor genannte Vorteile.

Das Tragsystem kann gegenüberliegend von der Tragarmgelenkvorrichtung ein weiteres Gelenk aufweisen, an welchem die wenigstens eine Strebe, insbesondere ein Ende der wenigstens einen Strebe, und die medizintechnische Einrichtung befestigt sind.

Gemäß einem Ausführungsbeispiel ist eine/die Strebe des Tragarms in zwei voneinander separaten Abschnitten der Schwenkachse abgestützt, wobei die Abschnitte jeweils in einem Gehäuse der Tragarmgelenkvorrichtung gelagert sind, wobei der Verstellbereich bzw. die Kavität lateral außen vom Gehäuse begrenzt ist. Hierdurch kann trotz eines maximal großen Verstellbereichs eine besonders stabile Anordnung sichergestellt werden.

Gemäß der Erfindung ist der Tragarm als Parallelogramm-Tragarm ausgebildet, wobei die Tragarmgelenkvorrichtung zwei Schwenkachsen jeweils für eine Strebe des Tragarms definiert, wobei die Stützachse oberhalb von beiden Schwenkachsen angeordnet ist und der Achsabstand zwischen der Stützachse und einer oberen der Schwenkachsen zum Einstellen einer minimalen Nutzlast minimierbar ist. Durch die Position der Stützachse im Verhältnis zu einer bzw. zu beiden Streben kann die Hebelwirkung und damit die Nutzlast eingestellt werden.

Die erfindungsgemäße Stativvorrichtung zur Anordnung im Operationssaal und zum örtlichen Verlagern wenigstens einer medizintechnischen Einrichtung im Operationssaal, umfasst wenigstens das erfindungsgemäße Tragsystem, wobei die wenigstens eine medizintechnische Einrichtung eine vordefinierte Masse aufweist und in vordefinierter Entfernung zur Tragarmgelenkvorrichtung an einem Tragarm des Tragsystems, insbesondere an einem Ende des Tragarms, gehalten ist, wobei die Tragarmgelenkvorrichtung auf eine Nutzlast entsprechend einer vom Tragarm aufzunehmenden Gewichtskraft der medizintechnischen Einrichtung eingestellt ist, indem eine Stützachse der Tragarmgelenkvorrichtung unabhängig von der Position einer Schwenkachse der Tragarmgelenkvorrichtung relativ zur Schwenkachse angeordnet/anordenbar ist, insbesondere in einem räumlich von den Schwenkachsen getrennt angeordneten Verstellbereich. Hierdurch ergeben sich bereits zuvor genannte Vorteile.

Die erfindungsgemäße Verwendung einer Tragarmgelenkvorrichtung zum Einstellen einer Nutzlast des medizintechnischen Tragsystems, wobei eine Stützachse der Tragarmgelenkvorrichtung in einem Verstellbereich angeordnet wird, welcher sich derart relativ zu wenigstens einer Schwenkachse der Tragarmgelenkvorrichtung erstreckt, dass die Stützachse oberhalb von der Position der Schwenkachse angeordnet ist, bevorzugt seitlich versetzt von der Schwenkachse.

In den nachfolgenden Zeichnungsfiguren wird die Erfindung anhand eines Ausführungsbeispiels näher erläutert. Es zeigen:
- Figur 1A, 1B: in teilgeschnittener und perspektivischer Ansicht ein Tragarmgelenk bzw. einen Tragarm gemäß dem Stand der Technik;
- Figuren 2A, 2B, 2C: in teilgeschnittener und perspektivischer Ansicht eine Tragarmgelenkvorrichtung bzw. eine Tragarmvorrichtung gemäß einem Ausführungsbeispiel der Erfindung, wobei ein Lagerelement in einer oberen Position angeordnet ist;
- Figuren 3A, 3B, 3C: in teilgeschnittener und perspektivischer Ansicht eine Tragarmgelenkvorrichtung bzw. eine Tragarmvorrichtung gemäß einem Ausführungsbeispiel der Erfindung mit dem Lagerelement in einer unteren Position;
- Figur 4: in teilgeschnittener Ansicht eine Tragarmvorrichtung mit einer Tragarmgelenkvorrichtung gemäß einem Ausführungsbeispiel der Erfindung mit dem Lagerelement in einer mittleren Position; und
- Figuren 5A, 5B: in teilgeschnittener und perspektivischer Ansicht Komponenten einer Stativvorrichtung mit einer medizintechnischen Einrichtung, einer Tragarmvorrichtung und einer Tragarmgelenkvorrichtung gemäß einem Ausführungsbeispiel der Erfindung.

Im Zusammenhang mit der Beschreibung der folgenden Figuren wird bei Bezugszeichen, falls sie in einzelnen Figuren nicht explizit erläutert werden, auf die weiteren Figuren verwiesen.

In der Figur 1A ist ein Tragarmgelenk 10a gemäß dem Stand der Technik gezeigt, insbesondere in Ausführung als Schweißkonstruktion. Ein Tragarm 20a ist mittels des Tragarmgelenks 10a an einer Spindel 4 in einem Drehlagerabschnitt 11 um eine Drehachse D drehgelagert. Das Tragarmgelenk 10a umfasst eine Einstellmechanik 12, 13, 14, 15, 16 zur Justage einer Vorspannung im Tragarm 20a.

Am Tragarmgelenk 10a greifen zwei Streben 21, 23 des Tragarms 20a an. Die obere Strebe 21 umfasst ein Hebellager 21.1 und eine Feder 21.2, insbesondere eine Druckfeder, welche Druck auf eine Gewindespindel ausübt, welche diese Druckkraft als Zugkraft auf einen Hebel überträgt. Das Hebellager 21.1 ist als Gleitlager ausgeführt und verbindet einen Hebel 13 mit einer Federspindel 21.3. Die Feder 21.2 erzeugt eine Gegenkraft zur Gewichtskraft bzw. Masse einer medizintechnischen Einrichtung (nicht dargestellt), die an einem Ende gegenüberliegend vom Tragarmgelenk 10a am Tragarm 20a befestigt ist. Die obere Strebe 21 kann als Federrohr beschrieben werden, welches eine Feder-Baugruppe aufnimmt und als Abstützung der gesamten auf den Tragarm von einer medizintechnischen Einrichtung ausgeübten Last dient.

Die obere Strebe 21 ist an einer oberen Schwenkachse 17.1a am Tragarmgelenk 10a gelagert, und die untere Strebe 23 ist in einer unteren Schwenkachse 17.2 am Tragarmgelenk 10a gelagert. Hierdurch kann eine Parallelogramm-Anordnung definiert werden, bei welcher beim Verschwenken des Tragarms bzw. der medizintechnischen Einrichtung nach oben oder nach unten Reaktionskräfte in der Feder 21.2 und im Hebel 13 hervorgerufen werden. Der Hebel 13 stützt die Parallelogramm-Anordnung in einem weiteren Lagerpunkt ab, nämlich an der Stützachse bzw. Lagerbockachse X.

Im Folgenden wird die Einstellmechanik beschrieben. Ein Lagerbock 12 lagert den Hebel 13 am Tragarmgelenk 10a und kann Federkräfte ins Tragarmgelenk 10a einleiten. Der Lagerbock 12 definiert die Position der Stützachse bzw. Lagerbockachse X, an welcher der Hebel 13 gelagert ist. Ein Gehäuse 18 des Tragarmgelenks wiederum kann die unterschiedlichen möglichen Positionen des Lagerbocks 12 vorgeben. Ein Abstand dz zwischen dem Lagerbock bzw. der Lagerbockachse X und der Schwenkachse 17.1 a ist einstellbar. Der maximal mögliche Abstand definiert einen Verstellbereich Vz, welcher durch das Tragarmgelenk vorgegeben ist. Der Verstellbereich Vz entspricht dabei einem Bewegungsbereich, in welchem das Lagerelement 12 bzw. der Lagerbock bzw. die Lagerbockachse X in relativem Abstand zur oberen Schwenkachse 17.1a positioniert werden kann. Der Verstellbereich Vz wird auch durch einen ersten Anschlag 19.1, an welchem das Lagerelement 12 mit minimalem Abstand zwischen den Achsen (Schwenkachse 17.1, 17.2 und Stützachse X) zur Anlage kommen kann, und einem diesem gegenüberliegenden zweiten Anschlag 19.2 definiert. Beide Anschläge 19.1, 19.2 werden vorliegend durch das Gehäuse 18 der Tragarmgelenkvorrichtung ausgebildet, könnten aber auch durch ein oder mehrere zusätzliche Bauteile verwirklicht werden. Der zweite Anschlag definiert eine Anschlagebene E. Das Einstellen des Abstandes dz und damit einhergehend der Nutzlast des Tragarms kann mittels Justagemitteln 14, insbesondere in Ausgestaltung als Stellschraube erfolgen. Eine Gegenschraube 15 kann die Justagemittel 14 dabei spielfrei lagern und sichern. Der Lagerbock 12 ist durch Führungsmittel 16, insbesondere Führungsbolzen geführt. In erster Linie nehmen die Führungsmittel 16 horizontal ausgerichtete Kräfte auf. In vertikaler Richtung ausgerichtete Kräfte werden, zumindest zu einem großen Teil, auf die Justagemittel 14 weitergeleitet. Aufgrund einer Verklemmung an den Führungsmitteln 16 kann nicht vermieden werden, dass auch teilweise vertikalen Kraftkomponenten auf die Führungsmittel 16 übertragen werden.

Das Lagerelement 12 weist einen Lagerfortsatz 12.2 auf, der in einer der wenigstens einen Schwenkachse 17.1, 17.2 abgewandten Richtung von einem zentralen Teil des Lagerelements 12 abragt, so dass die Stützachse X in ihrer maximal von der wenigstens einen Schwenkachse 17.1, 17.2 beabstandendeten Position Pₘₐₓ im Bereich der Anschlagebene E, insbesondere im Wesentlichen in der Anschlagebene E, liegt. Die Anschlagebene E ist dabei in einer durch den Verstellbereich Vz vorgegebenen Richtung maximal entfernt von der wenigstens einen Schwenkachse 17.1, 17.2.

Die Federvorspannung des Tragarms 20 und damit die Nutzlast kann werkseitig auf den Maximalwert für den entsprechenden (gewünschten) Nutzlastbereich eingestellt werden, z.B. 12-18kg. Dies kann in Hinblick auf eine Masse der medizintechnischen Einrichtung erfolgen. Eine Feinjustage der Nutzlast erfolgt durch Verschiebung des Lagerbocks 12 bzw. der Lagerbockachse X im Verstellbereich Vz, also von der (dargestellten) obersten Position nach unten bis zur oberen Schwenkachse 17.1a. Die Verschiebung erfolgt durch Drehen der Stellschraube 14. In Abhängigkeit von der Drehrichtung der Stellschraube 14 wandert der Lagerbock 12 nach oben bzw. unten und verändert damit den Winkel des Hebels 13 und dadurch den von der Feder 21.2 hervorgerufenen Effekt.

In der oberen Position des Lagerbocks 12 (maximaler Abstand dz, Pₘₐₓ) kann der Tragarm 20 dann die maximale Last von z.B. 18kg aufnehmen, und in der unteren Position des Lagerbocks 12 (dz=0), also in einer Position angrenzend an die obere Schwenkachse 17.1a, wird nur die minimale Last z.B. 12kg abgestützt. Der Lagerbock 12 kann dabei nicht an der oberen Schwenkachse 17.1a vorbei geführt werden, sondern nur bis zu einer Position angrenzend an die Schwenkachse. Mit diesem werkseitig vordefinierten Bereich einer Federvorspannung lassen sich Nutzlasten kleiner als den hier beispielhaft genannten 12kg nicht einstellen. Um kleinere Nutzlasten zu ermöglichen, ist eine andere werkseitig eingestellte Federvorspannung bzw. eine andere Feder 21.2 erforderlich.

In der Figur 1B ist die Anordnung der Stützachse bzw. Lagerbockachse X relativ zur oberen Schwenkachse 17.1a dargestellt. Die obere Schwenkachse 17.1a erstreckt sich dabei über die gesamte Länge zwischen zwei außenliegenden Gehäusewandungen 18.1, 18.2 des Tragarmgelenks.

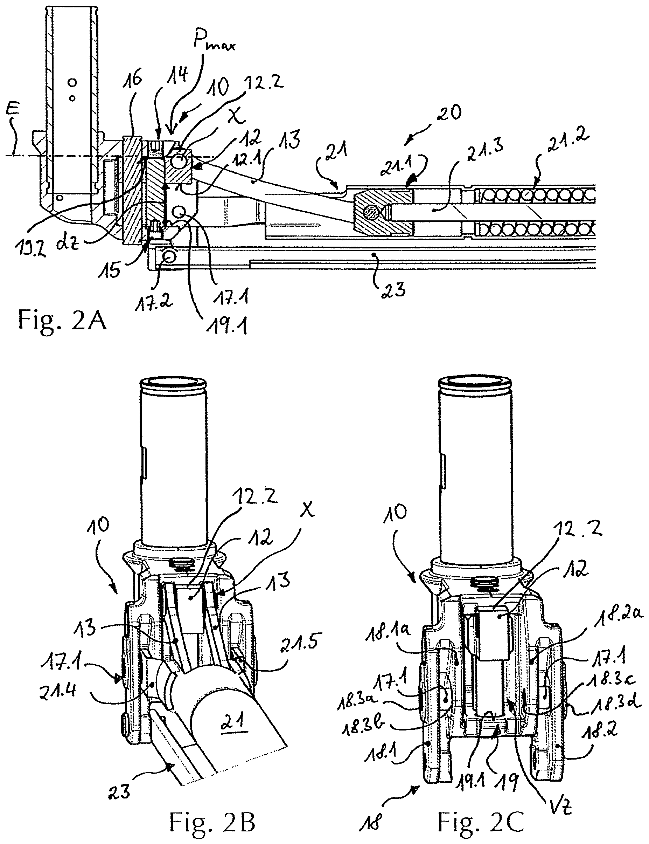

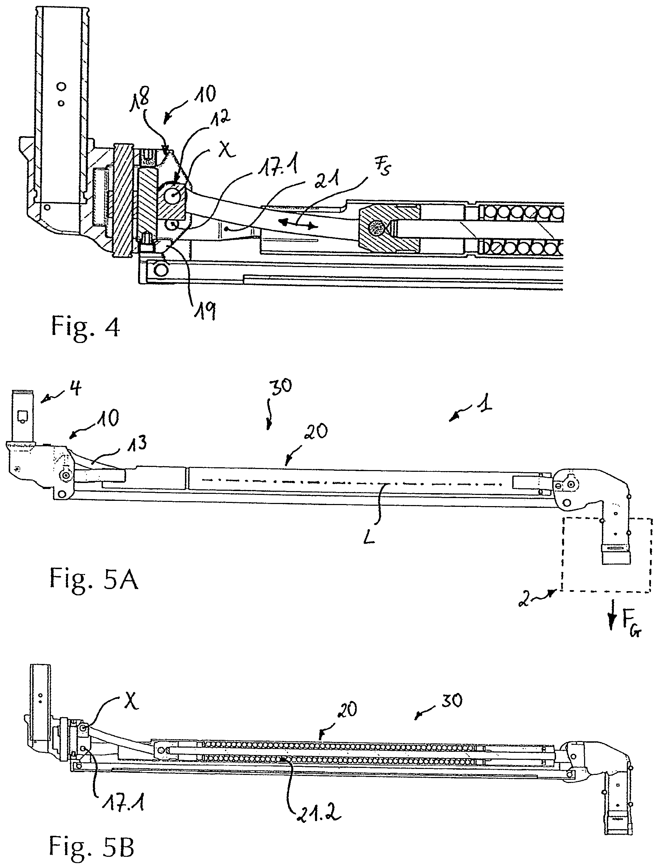

In den Figuren 2A, 2B, 2C ist eine Tragarmgelenkvorrichtung 10 gezeigt, welche einen vergleichsweise großen Verstellbereich für die Stützachse bzw. Lagerbockachse X und damit einen großen Verstellbereich für Nutzlasten liefert. Eine Feder 21.2, insbesondere eine Druckfeder, erzeugt Vorspannkräfte, die über die Federspindel 21.3 als Zugkräfte auf den Hebel 13 übertragen werden. Dazu kann die Druckfeder zwischen zwei Punkten im Federrohr 21 verspannt sein. Auf einer der Tragarmgelenkvorrichtung 10 zugewandten Seite werden Kräfte gegen eine Einschnürung im Federrohr ausgeübt, und auf der gegenüberliegenden Seite werden Kräfte gegen die Gewindespindel 21.3 ausgeübt, die durch die Feder 21.2 hindurch geht, und auf die eine Vorspannmutter zum (insbesondere werksseitigen) Einstellen der Vorspannung geschraubt ist, wie in Fig. 5B gezeigt. Dadurch kann mittels der Druckfeder 21.2 auf die Gewindespindel 21.3 eine Zugkraft ausgeübt werden, die über das Hebellager und den Hebel auf das Lagerelement/den Lagerbock 12 übertragen wird.

Auch bei diesem Ausführungsbeispiel wird der Verstellbereich Vz auch durch einen ersten Anschlag 19.1, an welchem das Lagerelement 12 mit minimalem Abstand zwischen den Achsen (Schwenkachse 17.1, 17.2 und Stützachse X) zur Anlage kommen kann, und einem diesem gegenüberliegenden zweiten Anschlag 19.2 definiert. Beide Anschläge 19.1, 19.2 werden vorliegend durch das Gehäuse 18 der Tragarmgelenkvorrichtung ausgebildet, könnten aber auch durch ein oder mehrere zusätzliche Bauteile verwirklicht werden. Der zweite Anschlag definiert eine Anschlagebene E. Das Lagerelement 12 weist ebenfalls wieder einen Lagerfortsatz 12.2 auf, der in einer der wenigstens einen Schwenkachse 17.1, 17.2 abgewandten Richtung von einem zentralen Teil des Lagerelements 12 abragt, so dass die Stützachse X in ihrer maximal von der wenigstens einen Schwenkachse 17.1, 17.2 beabstandendeten Position Pₘₐₓ im Bereich der Anschlagebene E, insbesondere im Wesentlichen in der Anschlagebene E, liegt. Die Anschlagebene E ist dabei in einer durch den Verstellbereich Vz vorgegebenen Richtung maximal entfernt von der wenigstens einen Schwenkachse 17.1, 17.2.

Die Tragarmgelenkvorrichtung 10 ist als Gussteil ausgeführt, insbesondere aus Aluminium. Das Guss-Gehäuse 18 weist zusätzlich zu den Wandungen 18.1, 18.2 auch Laschen 18.1a, 18.2a auf. Die obere Schwenkachse 17.1 erstreckt sich nicht vollständig zwischen den Wandungen 18.1, 18.2, sondern ist in zwei einzelne, separate Abschnitte unterteilt, welche sich jeweils zwischen einer der Wandungen 18.1, 18.2 und der korrespondierenden Lasche 18.1a, 18.2a erstrecken. Jeder Schwenkachsen-Abschnitt ist nur in einer der Wandungen 18.1, 18.2 gelagert. Jeder Schwenkachsen-Abschnitt ist in der korrespondierenden Lasche 18.1 a, 18.2a gelagert.

Auch bei dieser Anordnung kann die Nutzlast des Tragarms durch Verschieben des Lagerbocks 12 entlang des Führungsbolzens 16 mittels der Stellschraube 14 eingestellt werden. Jedoch kann ein maximal großer Verstellbereich Vz bereitgestellt werden, so dass der Tragarm für ein breiteres Spektrum von Nutzlasten eingerichtet ist. Die obere Schwenkachse 17.1 ist hierzu als geteilte Achse ausgeführt. Hierdurch kann der Lagerbock 12 in der unteren Position zwischen die einzelnen Abschnitte/Teile der Achse 17.1 verfahren werden, ohne mit der Achse 17.1 zu kollidieren. In der dargestellten Ausführung ist der Bewegungsbereich des Lagerbocks 12 allenfalls nur durch die innere Kontur des Tragarmgelenks 20 beschränkt. Der Lagerbock 12 kann über einen vergleichsweise großen Verstellbereich Vz verfahren werden und damit einen vergleichsweise großen Einstellbereich der Nutzlast sicherstellen. Das ermöglicht beispielsweise, mit einer Werkseinstellung der Federvorspannung, die auf das Maximum von z.B. 21kg eingestellt wird, den Nutzlastbereich des Tragarms zwischen z.B. 1,5kg und 21kg direkt mittels der Justagemittel 14 am Einsatzort einzustellen oder nachzujustieren.

In der Figur 2A ist der Abstand dz maximal groß. Das Lagerelement 12 ist in einer obersten Position angeordnet.

Die Anordnung der Stellschraube 14 zwischen dem Führungsbolzen 16 und der Stützachse X kann eine vorteilhafte Spannungsverteilung im Gussgehäuse 18 sicherstellen.

Die Ausrichtung der Stellschraube 14 und des Führungsbolzens 16 ist vertikal bzw. in orthogonaler Richtung zu in horizontaler Lage ausgerichteten Streben 21, 23. Hierdurch kann ein besonders großer Schwenkbereich der Tragarmvorrichtung 20 ermöglicht werden. Die Kinematik des Parallelogramm-Tragarms 20 kann dabei auf zweckdienliche Weise mit der Tragarmgelenkvorrichtung gekuppelt werden. Ferner kann bei dieser vertikalen Ausrichtung von Stellschraube 14 und Führungsbolzen 16 eine Bearbeitung der Tragarmgelenkvorrichtung 10 auf einfache Weise erfolgen.

Der erzielbare Schwenkbereich beträgt aus der horizontalen Anordnung heraus beispielsweise 45° nach oben und -70° nach unten.

Das Tragarmgelenk 10 kann beispielsweise als Aluminium-Gusskonstruktion ausgeführt sein. Die Ausgestaltung als Gussteil liefert den Vorteil einer vergleichsweise einfachen, robusten Lagerung der beiden Abschnitte der oberen Schwenkachse 17.1. Das Guss-Gehäuse 18 kann auch bei einer geteilten Schwenkachse 17.1 mit hoher Torsions- und Biegesteifigkeit bereitgestellt werden. Eine Schweißkonstruktion hingegen müsste auf aufwändige Weise abgestützt werden.

In Figur 2B ist gezeigt, dass die obere Strebe 21 zwei Fortsätze 21.4, 21.5 aufweist, welche jeweils separat an einem der separaten Achsabschnitte der oberen Schwenkachse 17.1 gelagert sind, insbesondere jeweils zwischen zwei Gehäusefortsätzen.

Wie in Figur 2C dargestellt, definiert das Gehäuse 18 vier Lagerpunkte 18.3a, 18.3b, 18.3c, 18.3d, wobei jede Halbachse bzw. jeder Achsabschnitt der oberen Schwenkachse 17.1 in zwei der Lagerpunkte gelagert ist. Die Lagerpunkte 18.3a, 18.3b, 18.3c, 18.3d können zusammen mit dem jeweiligen Achsabschnitt eine Übergangspassung bilden.

In Figur 2C ist ferner der Verstellbereich Vz gezeigt, welcher sich nach unten bis hin zu einem Anschlag 19 am Gehäuse 18 erstreckt. Der Lagerbock 12 kann an einer ebenen Anschlagsfläche 19.1 zur Anlage kommen (unterste Position).

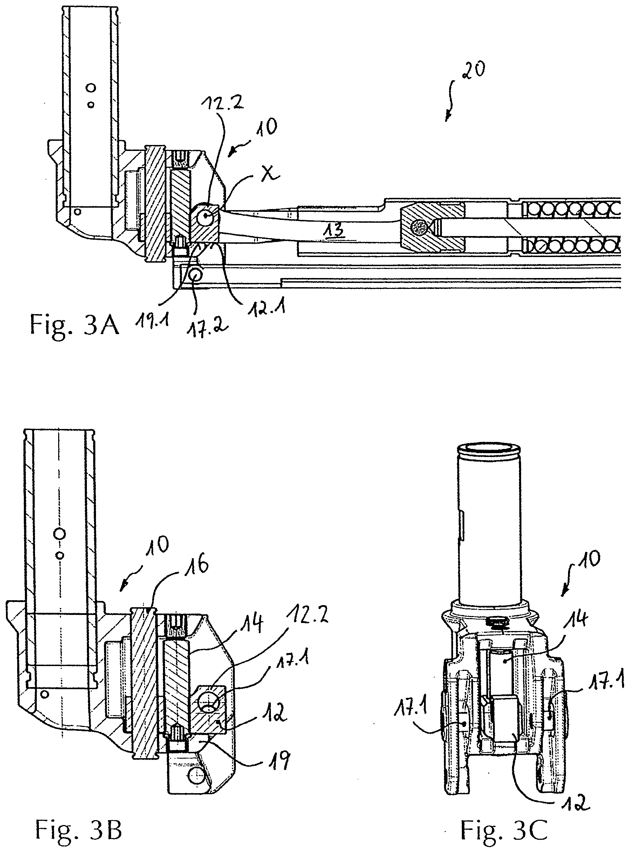

In den Figuren 3A, 3B, 3C ist das Lagerelement 12 bzw. die Stützachse X in einer untersten Position gezeigt, entsprechend der kleinstmöglichen Nutzlast der Tragarmvorrichtung 20. Die ebene Unterseite 12.1 des Lagerelements 12 liegt am ebenen Flächenabschnitt 19.1 des Gehäuses 18 bzw. Anschlags 19 an. Der Abstand dz ist NULL. Aus der Figur 3B geht hervor, dass die Anordnung des Lagerelements 12 unabhängig von der Position der oberen Schwenkachse 17.1 ist. Das Lagerelement 12 ist an der Schwenkachse 17.1 vorbei geführt und überlappt mit dieser. Die Stützachse ist dann nahezu auf der Höhe der Schwenkachse 17.1 angeordnet. Wahlweise kann die Stützachse auch noch weiter unten angeordnet sein, jedoch wird dann der Effekt der Feder immer geringer.

In der Figur 4 ist eine mittlere Position der Stützachse X mittig im Verstellbereich Vz gezeigt. Der Lagerbock 12 ist zumindest annähernd mittig zwischen dem unteren Anschlag 19 und einem entsprechenden oberen Anschlag im Gehäuse 18 angeordnet. Der Lagerbock 12 bzw. die Stützachse X kann unabhängig von der Position der oberen Schwenkachse 17.1 angeordnet werden. Stützkräfte FS, vornehmlich Zugkräfte, können im Hebel 13 weitergeleitet werden, so dass der Tragarm 20 in einer gewünschten Ausrichtung, z.B. horizontal, stabilisiert werden kann. In der gezeigten Anordnung des Hebels 13 bewirkt eine Kraft FS mit vordefiniertem Betrag einen mittleren Effekt, da der Hebelarm zwischen der Stützachse X und der oberen Schwenkachse 17.1 mittelgroß eingestellt ist.

In den Figuren 5A, 5B sind einzelne Komponenten einer Stativvorrichtung 1 gezeigt, umfassend eine medizintechnische Einrichtung 2, ein Tragsystem 30 mit wenigstens einem sich entlang einer Längsachse L erstreckenden Tragarm 20 und wenigstens einer Tragarmgelenkvorrichtung 10 sowie einer Spindel bzw. einem Zapfen 4.

Die medizintechnische Einrichtung 2 übt eine Gewichtskraft FG aus, welche über den Hebel 13 und die Stützachse in der Tragarmgelenkvorrichtung 10 aufgenommen und auf die Spindel 4 übertragen wird. In der gezeigten Anordnung der Stützachse X mit maximalem Abstand dz relativ zur oberen Schwenkachse kann die größte Nutzlast abgestützt werden. Die Spindel 4 kann z.B. in einer Buchse oder einem Deckenflansch gelagert sein.

Die zugrundeliegende Idee kann mit den folgenden Worten zusammengefasst werden. Eine Nutzlast eines Tragarmgelenks oder eines Tragsystems kann dadurch in einem breiten Spektrum eingestellt werden, dass eine Stützachse des Tragarmgelenks unabhängig von der Position einer Schwenkachse relativ zu dieser Schwenkachse in einem vordefinierbaren Abstand bzw. mit vordefinierbarem Hebelarm anordenbar ist, insbesondere in einem vergleichsweise kleinen Abstand. Die Stützachse kann in einem Abstützelement bzw. Lagerbock angeordnet sein, und das Abstützelement kann an der Schwenkachse vorbei verlagert werden, so dass das Abstützelement unabhängig von der Position der Schwenkachse geometrisch ausgestaltet und angeordnet werden kann. Ein solches Tragarmgelenk kann besonders stabil, struktursteif und robust ausgeführt werden und einen in Bezug auf die Abmessungen des Tragarmgelenks maximal großen Verstellbereich bereitstellen. Letztendlich kann dabei mit einem vergleichsweise kleinen, kompakten und sehr struktursteifen Tragarmgelenk ein breites Nutzlast-Spektrum abgedeckt werden. Beispielsweise kann dann auch die Varianten-Anzahl für dieses Tragarmgelenk verringert werden.

### Bezugszeichenliste

- 1: Stativvorrichtung, insbesondere Deckenstativvorrichtung
- 2: medizintechnische Einrichtung
- 4: Spindel bzw. Zapfen

- 10a: Tragarmgelenk gemäß dem Stand der Technik
- 10: Tragarmgelenkvorrichtung
- 11: Drehlagerabschnitt
- 12: Lagerelement/Abstützelement, insbesondere Lagerbock
- 12.1: ebene Unterseite des Lagerelements
- 12.2: Lagerfortsatz des Lagerelements/Lagerbocks
- 13: Hebel
- 14: Justagemittel, insbesondere Stellschraube
- 15: Gegenschraube
- 16: Führungsmittel, insbesondere Führungsbolzen
- 17.1, 17.2, 17.1a: Schwenkachse für Strebe
- 18: Gehäuse
- 18.1, 18.2: Gehäusewandung
- 18.1a, 18.2a: Gehäuselasche
- 18.3a, 18.3b, 18.3c, 18.3d: Lagerpunkte am Gehäuse
- 19: Anschlag bzw. Absatz oder Vorsprung
- 19.1: ebener Flächenabschnitt des Anschlags
- 19.2: zweiter Anschlag
- 20a: Tragarm gemäß dem Stand der Technik
- 20: Tragarmvorrichtung (Federarm, Ausleger)
- 21: erste Strebe, insbesondere Federrohr
- 21.1: Hebellager in erster Strebe
- 21.2: Feder
- 21.3: Federspindel
- 21.4, 21.5: Strebenfortsatz
- 23: zweite Strebe
- 30: Tragsystem

- dz: einstellbarer Abstand zwischen Lagerbockachse und Schwenkachse
- D: Drehachse für Spindel, insbesondere vertikal ausgerichtete Hochachse
- E: Anschlagebene (des zweiten Anschlags)
- FG: Gewichtskraft der medizintechnischen Einrichtung
- FS: Stützkraft (Zug- oder Druckkraft)
- L: Längsachse des Tragarms
- Vz: Verstellbereich
- X: Stützachse bzw. Lagerbockachse

## Patentansprüche

1. Tragsystem für eine Stativvorrichtung (1) zur Anordnung im Operationssaal und zum örtlichen Verlagern einer medizintechnischen Einrichtung (2) mit vordefinierter Masse, umfassend
- wenigstens einen Tragarm (20) zum Halten der medizintechnischen Einrichtung mit wenigstens einer jeweils in einer Schwenkachse (17.1, 17.2) gelagerten Strebe (21, 23), wobei die Strebe mittels eines Hebels (13) zusätzlich an einer Stützachse (X) abgestützt ist; und
- wenigstens eine Tragarmgelenkvorrichtung (10) für den Tragarm (20) einer Stativvorrichtung (1) zur Anordnung im Operationssaal und zum örtlichen Verlagern einer am Tragarm gehaltenen medizintechnischen Einrichtung (2), wobei die Tragarmgelenkvorrichtung (10) zum Einstellen einer Nutzlast entsprechend einer vom Tragarm aufzunehmenden Gewichtskraft der medizintechnischen Einrichtung (2) eingerichtet ist, umfassend:
- wenigstens eine Schwenkachse (17.1, 17.2) jeweils zum Lagern wenigstens einer Strebe (21, 23) des Tragarms;
- eine Stützachse (X) zum Abstützen eines Hebels (13) eingerichtet zum Übertragen von den Tragarm haltenden Kräften zwischen der Strebe (21) und der Tragarmgelenkvorrichtung;
wobei ein Abstand (dz) zwischen den Achsen zum Einstellen der Nutzlast in einem Verstellbereich (Vz) justierbar ist;
wobei die Größe und/oder Erstreckung des Verstellbereichs (Vz) unabhängig von der Position der Schwenkachse (17.1) ist und
wobei die Schwenkachse (17.1) in Abschnitte unterteilt ist, sodass die relative Position der Stützachse (X) relativ zur Schwenkachse (17.1) unabhängig von der Position der Schwenkachse (17.1) oder des Hebels (13) einstellbar ist, insbesondere der Achsabstand (dz) minimierbar ist, wobei die Schwenkachse (17.1) in wenigstens zwei Abschnitte geteilt ist, zwischen welchen eine Kavität gebildet ist, wobei der Verstellbereich (Vz) in der Kavität angeordnet ist,
wobei die Strebe (21) des Tragarms an wenigstens einem Abschnitt an der Schwenkachse (17.1) neben der von der Tragarmgelenkvorrichtung definierten Kavität gelagert ist, in welchem die Stützachse (X) angeordnet ist und in unterschiedlichen Positionen im Verstellbereich (Vz) anordenbar ist, wobei die Schwenkachse (17.1) räumlich vom Verstellbereich entkoppelt ist,
wobei der Tragarm als Parallelogramm-Tragarm ausgebildet ist und die Tragarmgelenkvorrichtung (10) zwei Schwenkachsen (17.1, 17.2) jeweils für eine der Streben (21, 23) des Tragarms definiert, wobei die Stützachse (X) oberhalb von beiden Schwenkachsen angeordnet ist und der Achsabstand zwischen der Stützachse und einer oberen Schwenkachse zum Einstellen einer minimalen Nutzlast minimierbar ist.

2. Tragsystem nach Anspruch 1, wobei die Schwenkachse (17.1) derart relativ zur Stützachse (X) angeordnet und/oder dimensioniert ist, dass Schwenk- und Stützachse nicht überlappen, insbesondere in Bezug auf deren Achsrichtung.

3. Tragsystem nach einem der vorhergehenden Ansprüche, wobei die Schwenkachse (17.1) in wenigstens zwei Abschnitte geteilt ist, zwischen welchen eine Kavität, insbesondere zentrisch in der Tragarmgelenkvorrichtung gebildet ist, wobei der Verstellbereich (Vz) in der Kavität angeordnet ist, wobei die Kavität zur teilweisen Aufnahme des Hebels zwischen die Achsenabschnitte oder neben einen jeweiligen Achsenabschnitt eingerichtet ist.

4. Tragsystem nach einem der vorhergehenden Ansprüche, ferner umfassend:
- ein Lagerelement (12), insbesondere Lagerbock, welches die Position der Stützachse (X) definiert und an den Hebel (13) kuppelbar ist; und
- Justagemittel (14) zum Einstellen des Achsabstands im Verstellbereich, wobei das Lagerelement an die Justagemittel gekuppelt ist.

5. Tragsystem nach Anspruch 4, wobei die Justagemittel (14) eingerichtet sind, das Lagerelement (12) derart an der Schwenkachse (17.1) vorbei zu führen und/oder derart in der Kavität neben der Schwenkachse zu positionieren, dass der Achsabstand minimiert ist.

6. Tragsystem nach einem der vorhergehenden Ansprüche 4 oder 5, wobei die Tragarmgelenkvorrichtung, insbesondere ein Gehäuse (18) der Tragarmgelenkvorrichtung, einen Anschlag (19) definiert, an welchem das die Stützachse (X) definierendes Lagerelement (12) in einer Anordnung mit minimalem Achsabstand zur Anlage kommen kann, wobei der Anschlag (19) bevorzugt unterhalb von der Schwenkachse (17.1) angeordnet ist.

7. Tragsystem nach einem der vorhergehenden Ansprüche, wobei wenigstens ein separater Abschnitt der Schwenkachse (17.1) separat in einer Gehäusewandung (18.1, 18.2) und bevorzugt auch in einer korrespondierenden Gehäuselasche (18.1a, 18.2a) gelagert ist, welche jeweils an einer Seite vom Verstellbereich (Vz) angeordnet ist.

8. Tragsystem nach einem der vorhergehenden Ansprüche 4 bis 7, ferner umfassend Führungsmittel (16) eingerichtet zum Führen des die Stützachse (X) definierendes Lagerelements (12) entlang einer vordefinierten Bewegungsbahn im Verstellbereich, insbesondere entlang einer Geraden, wobei zum Einstellen des Achsabstands vorgesehene Justagemittel bevorzugt zwischen den Führungsmitteln und der Schwenkachse (17.1) angeordnet sind.

9. Tragsystem nach einem der vorhergehenden Ansprüche, wobei die Tragarmgelenkvorrichtung (10) ein Gussteil ist, insbesondere aus Aluminium.

10. Tragsystem nach einem der vorhergehenden Ansprüche 6 bis 9, wobei das Gehäuse (18) der Tragarmgelenkvorrichtung einen dem Anschlag (19) gegenüberliegenden zweiten Anschlag (19.2) aufweist, der eine Anschlagebene (E) definiert, und wobei das Lagerelement (12) einen Lagerfortsatz (12.2) aufweist, der in einer der wenigstens einen Schwenkachse (17.1, 17.2) abgewandten Richtung von einem zentralen Teil des Lagerelements (12) abragt, so dass die Stützachse (X) in ihrer maximal von der wenigstens einen Schwenkachse (17.1, 17.2) beabstandeten Position (Pₘₐₓ) im Bereich der Anschlagebene (E) liegt.

11. Tragsystem nach einem der vorhergehenden Ansprüche, wobei die Strebe (21) des Tragarms in zwei voneinander separaten Abschnitten der Schwenkachse (17.1) abgestützt ist, wobei die Abschnitte jeweils in einem Gehäuse (18) der Tragarmgelenkvorrichtung (10) gelagert sind, wobei der Verstellbreich lateral außen vom Gehäuse begrenzt ist.

12. Stativvorrichtung (1) zur Anordnung im Operationssaal und zum örtlichen Verlagern wenigstens einer medizintechnischen Einrichtung (2) im Operationssaal, umfassend wenigstens ein Tragsystem nach einem der vorhergehenden Ansprüche,
wobei die wenigstens eine medizintechnische Einrichtung (2) eine vordefinierte Masse aufweist und an einem Tragarm (20) des Tragsystems, insbesondere an einem Ende des Tragarms, gehalten ist, wobei die Tragarmgelenkvorrichtung (10) auf eine Nutzlast entsprechend einer vom Tragarm aufzunehmenden Gewichtskraft der medizintechnischen Einrichtung (2) eingestellt ist, indem eine Stützachse (X) der Tragarmgelenkvorrichtung (10) unabhängig von der Position einer Schwenkachse (17.1, 17.2) der Tragarmgelenkvorrichtung (10) relativ zur Schwenkachse anordenbar ist, insbesondere in einem räumlich von den Schwenkachsen getrennt angeordneten Verstellbereich (Vz).

13. Verwendung eines Tragsystems nach einem der vorhergehenden Ansprüche 1 bis 11 zum Einstellen einer Nutzlast des medizintechnischen Tragsystems, wobei eine Stützachse (X) der wenigstens einen zum Tragsystem gehörigen Tragarmgelenkvorrichtung (10) in einem Verstellbereich (Vz) angeordnet wird, welcher sich derart relativ zu wenigstens einer Schwenkachse (17.1, 17.2) der Tragarmgelenkvorrichtung erstreckt, dass die Stützachse räumlich unabhängig von der Position der Schwenkachse oberhalb von der Schwenkachse angeordnet ist, bevorzugt seitlich versetzt von der Schwenkachse.

## Claims

1. Carrier system for a stand device (1) for arranging in the operating room and for displacing a medical device (2) having a predefined mass, comprising
- at least one carrier arm (20) for holding a medical device with at least one strut (21, 23) supported in a respective pivot axis (17.1, 17.2), wherein the strut is additionally held by means of a lever (13) to a support axis (X); and
- at least one carrier arm joint device (10) for the carrier arm (20) of the stand device (1) for arranging in the operating room and for displacing a medical device (2) held on the carrier arm, wherein the carrier arm joint device (10) is configured for setting a payload corresponding to a weight of the medical device (2) to be taken up by the carrier arm, comprising:
- at least one pivot axis (17.1, 17.2) for mounting at least one strut (21, 23) of the carrier arm, respectively;
- a support axis (X) to support a lever (13) configured to transfer forces holding the carrier arm between the strut (21) and the carrier arm joint device;
wherein a distance (dz) between the axes is adjustable within an adjustment range (Vz) in order to set the payload;
wherein the size and/or the extension of the adjustment range (Vz) is independent of the position of the pivot axis (17.1), and
wherein the pivot axis (17.1) is partitioned in portions in a way that the relative position of the support axis (X) in relation to the pivot axis (17.1) may be set independent of the position of the pivot axis (17.1) or the lever (13), in particular the axial distance (dz) may be minimized, wherein the pivot axis (17.1) is partitioned in at least two partitions, between which a cavity is formed, wherein the adjustment range (Vz) is arranged in the cavity, wherein the strut (21) of the carrier arm is supported on at least one portion on the pivot axis (17.1) adjacent to the cavity defined by the carrier arm joint device in which the support axis (X) is arranged and is arrangeable in different positions within the adjustment range (Vz), wherein the pivot axis (17.1) is spatially decoupled from the adjustment range,
wherein the carrier arm is adapted as a parallelogram-like carrier arm, and wherein the carrier arm joint device (10) defines two pivot axes (17.1, 17.2) for one of the struts (21, 23) of the carrier arm, respectively, wherein the support axis (X) is arranged above both pivot axes and the axial distance between the support axis and an upper pivot axis may be minimized in order to set the minimum payload.

2. Carrier system according to claim 1, wherein the pivot axis (17.1) is arranged and/or dimensioned relative to the support axis (X) such that pivot and support axis do not overlap, in particular regarding the axial orientation thereof.

3. Carrier system according to any one of the preceding claims, wherein the pivot axis (17.1) is partitioned in at least two partitions, between which a cavity is formed, in particular centered in the carrier arm joint device, wherein the adjustment range (Vz) is arranged in the cavity, wherein the cavity is configured to partially accommodate the lever between the axial portions or adjacent to a respective axial portion.

4. Carrier system according to any one of the preceding claims, further comprising:
- a bearing elements (12), in particular a bearing block, which defines the position of the support axis (X) and is coupable to the lever (13); and
- adjustment means (14) for setting the axial distance in the adjustment range, wherein the bearing element is coupled to the adjustment means.

5. Carrier system according to claim 4, wherein the adjustment means (14) are configured to guide the bearing element (12) past to the pivot axis (17.1) and/or to position it in the cavity adjacent to the pivot axis such that the axial distance is minimized.

6. Carrier system according to any one of preceding claims 4 or 5, wherein the carrier arm joint device, in particular a casing (18) of the carrier arm joint device, defines a stop (19), on which the bearing element (12) defining the support axis (X) may abut in an arrangement with a minimum axial distance, wherein the stop (19) is preferably arranged below the pivot axis (17.1).

7. Carrier system according to any one of the preceding claims, wherein at least one separate portion of the pivot axis (17.1) is separately supported in a casing wall (18.1, 18.2) and preferably also in a corresponding casing tongue (18.1a, 18.2a), which is arranged on a respective side of the adjustment range (Vz).

8. Carrier system according to any one of claims 4 to 7, further comprising guiding means (16) configured to guide the bearing element (12) defining the support axis (X) along a predefined movement path in the adjustment range, in particular along a straight line, wherein the adjustment means provided for adjustment of the axial distance are preferably positioned between the guiding means and the pivot axis (17.1).

9. Carrier system according to anyone of the preceding claims, wherein the carrier arm joint device (10) is a cast part, in particular made from aluminium.

10. Carrier system according to any one of claims 6 to 9, wherein the casing (18) of the carrier arm joint device comprises a second stop (19.2) opposite to the stop (19) which defines an abutment plane (E), and wherein the bearing element (12) comprises a bearing extension (12.2) which protrudes from a central part of the bearing element (12) in a direction opposite to the at least one pivot axis (17.1, 17.2), thus the support axis (X) is positioned in the position (Pmax) thereof, spaced apart from the at least one pivot axis (17.1, 17.2) in the maximum distance thereto, in the region of the abutment plane (E).

11. Carrier system according to any one of the preceding claims, wherein the strut (21) of the carrier arm is supported in two separate portions of the pivot axis (17.1), wherein the portions are supported in a casing (18) of the carrier arm joint device (10) respectively, wherein the adjustment range is limited by the casing laterally outside.

12. A stand device (1) for arranging in the operating room and for displacing at least one medical device (2) in the operating room, comprising at least one carrier system according to any one of the preceeding claims,
wherein the at least one medical device (2) comprises a predefined mass and is held on a carrier arm (20) of the carrier system, in particular on an end of the carrier arm, wherein the carrier arm joint device (10) is set to a payload corresponding to a weight of the medical device (2) to be taken up by the carrier arm, wherein a support axis (X) of the carrier arm joint device (10) is arrangeable independent of the position of the pivot axis (17.1, 17.2) of the carrier arm joint device (10) in relation to the pivot axis, in particular in an adjustment range (Vz) spatially separated from the pivot axes.

13. A use of the carrier system according to any one of preceding claims 1 to 11, for setting a payload of a medical carrier system, wherein a support axis (X) of the at least one carrier arm joint device (10) belonging to the carrier system is arranged in an adjustment range (Vz), which extends in relation to at least one pivot axis (17.1, 17.2) of the carrier arm joint device such that the support axis is arranged spatially independent of the position of the pivot axis above the pivot axis, preferably laterally offset of the pivot axis.

## Revendications

1. Système de support pour un dispositif à pied (1) destiné à être disposé dans la salle d'opération et à déplacer localement un dispositif médical (2) avec une masse prédéfinie, comprenant
- au moins un bras de support (20) pour maintenir le dispositif médical avec au moins un montant (21, 23) monté respectivement dans un axe de pivotement (17.1, 17.2), le montant étant en outre appuyé sur un axe d'appui (X) au moyen d'un levier (13) ; et
- au moins un dispositif d'articulation de bras de support (10) pour le bras de support (20) d'un dispositif à pied (1) destiné à être disposé dans la salle d'opération et à déplacer localement un dispositif médical (2) maintenu sur le bras de support, le dispositif d'articulation de bras de support (10) étant conçu pour régler une charge utile correspondant à une force de poids du dispositif médical (2) à absorber par le bras de support, comprenant :
- au moins un axe de pivotement (17.1, 17.2) destiné à monter respectivement au moins un montant (21, 23) du bras de support ;
- un axe d'appui (X) destiné à appuyer un levier (13) conçu pour transférer des forces maintenant le bras de support entre le montant (21) et le dispositif d'articulation de bras de support ;
un entraxe (dz) entre les axes de réglage de la charge utile étant réglable dans une zone de réglage (Vz) ;
la taille et/ou l'étendue de la zone de réglage (Vz) étant indépendante de la position de l'axe de pivotement (17.1) et
l'axe de pivotement (17.1) étant divisé en sections de sorte que la position relative de l'axe d'appui (X) par rapport à l'axe de pivotement (17.1) peut être réglée indépendamment de la position de l'axe de pivotement (17.1) ou du levier (13), en particulier l'entraxe (dz) peut être minimisé, l'axe de pivotement (17.1) étant divisé en au moins deux sections, entre lesquelles une cavité est formée, la zone de réglage (Vz) étant disposée dans la cavité,
le montant (21) du bras de support étant monté sur au moins une section sur l'axe de pivotement (17.1) à côté de la cavité définie par le dispositif d'articulation de bras de support, dans laquelle l'axe d'appui (X) est disposé et peut être disposé dans différentes positions dans la zone de réglage (Vz), l'axe de pivotement (17.1) étant découplé spatialement de la plage de réglage,
le bras de support étant conçu comme un bras de support en parallélogramme et le dispositif d'articulation de bras de support (10) définissant deux axes de pivotement (17.1, 17.2) respectivement pour l'un des montants (21, 23) du bras de support, l'axe d'appui (X) étant disposé au-dessus des deux axes de pivotement et l'entraxe entre l'axe d'appui et un axe de pivotement supérieur peut être minimisé pour régler une charge utile minimale.

2. Système de support selon la revendication 1, dans lequel l'axe de pivotement (17.1) est disposé et/ou dimensionné par rapport à l'axe d'appui (X) de telle sorte que les axes de pivotement et d'appui ne se chevauchent pas, notamment en ce qui concerne leur direction axiale.

3. Système de support selon l'une des revendications précédentes, dans lequel l'axe de pivotement (17.1) est divisé en au moins deux sections, entre lesquelles une cavité est formée, en particulier au centre du dispositif d'articulation de bras de support, la zone de réglage (Vz) étant disposée dans la cavité, la cavité étant conçue pour loger partiellement le levier entre les sections d'axe ou à côté d'une section d'axe respective.

4. Système de support selon l'une des revendications précédentes, comprenant en outre :
- un élément de palier (12), en particulier un bloc de palier, qui définit la position de l'axe d'appui (X) et peut être couplé au levier (13) ; et
- des moyens de réglage (14) pour régler l'entraxe dans la plage de réglage, l'élément de palier étant couplé aux moyens de réglage.

5. Système de support selon la revendication 4, dans lequel les moyens de réglage (14) sont conçus pour guider l'élément de palier (12) sur l'axe de pivotement (17.1) et/ou pour le positionner dans la cavité à côté de l'axe de pivotement de manière à ce que l'entraxe est minimisé.

6. Système de support selon l'une des revendications précédentes 4 ou 5, dans lequel le dispositif d'articulation de bras de support, en particulier un boîtier (18) du dispositif d'articulation de bras de support définit une butée (19) sur laquelle l'élément de palier (12) définissant l'axe d'appui (X) peut venir s'appuyer dans un agencement à entraxe minimal, la butée (19) étant de préférence disposée en dessous de l'axe de pivotement (17.1).

7. Système de support selon l'une des revendications précédentes, dans lequel au moins une section de séparation de l'axe de pivotement (17.1) est montée séparément dans une paroi de boîtier (18.1, 18.2) et de préférence également dans un support de boîtier correspondant (18.1a, 18.2a), qui est disposé respectivement sur un côté de la zone de réglage (Vz).

8. Système de support selon l'une des revendications précédentes 4 à 7, comprenant en outre des moyens de guidage (16) conçus pour guider l'élément de palier (12) définissant l'axe d'appui (X) le long d'une trajectoire de déplacement prédéfinie dans la zone de réglage, en particulier le long d'une ligne droite, des moyens de réglage prévus pour régler l'entraxe étant de préférence disposés entre les moyens de guidage et l'axe de pivotement (17.1).

9. Système de support selon l'une des revendications précédentes, dans lequel le dispositif d'articulation de bras de support (10) est une pièce moulée, en particulier en aluminium.

10. Système de support selon l'une des revendications précédentes 6 à 9, dans lequel le boîtier (18) du dispositif d'articulation de bras de support présente une seconde butée (19.2) située à l'opposé de la butée (19) et définissant un plan de butée (E), et dans lequel l'élément de palier (12) présente une extension de palier (12.2) qui fait saillie depuis une partie centrale de l'élément de palier (12) dans une direction opposée à l'au moins un axe de pivotement (17.1, 17.2), de sorte que l'axe d'appui (X) se trouve dans la zone du plan de butée (E) dans sa position (Pₘₐₓ) écartée au maximum de l'au moins un axe de pivotement (17.1,17.2).

11. Système de support selon l'une des revendications précédentes, dans lequel le montant (21) du bras de support est appuyé dans deux sections séparées l'une de l'autre de l'axe de pivotement (17.1), les sections étant chacune montées dans un boîtier (18) du dispositif d'articulation de bras de support (10), la zone de réglage étant délimitée latéralement à l'extérieur par le boîtier.

12. Dispositif à pied (1) destiné à être disposé dans la salle d'opération et à déplacer localement au moins un dispositif médical (2) dans la salle d'opération, comprenant au moins un système de support selon l'une des revendications précédentes,
l'au moins un dispositif médical (2) ayant une masse prédéfinie et étant maintenu sur un bras de support (20) du système de support, en particulier sur une extrémité du bras de support, le dispositif d'articulation de bras de support (10) étant réglé sur une charge utile correspondant à une force de poids du dispositif médical (2) devant être absorbée par le bras de support en ce qu'un axe d'appui (X) du dispositif d'articulation du bras de support (10) peut être disposé indépendamment de la position d'un axe de pivotement (17.1, 17.2) du dispositif d'articulation de bras de support (10) par rapport à l'axe de pivotement, en particulier dans une zone de réglage (Vz) spatialement séparée des axes de pivotement.

13. Utilisation d'un système de support selon l'une des revendications précédentes 1 à 11 pour le réglage d'une charge utile du système de support médical,
un axe d'appui (X) de l'au moins un dispositif d'articulation de bras de support (10) appartenant au système de support étant disposé dans une zone de réglage (Vz), qui s'étend par rapport à au moins un axe de pivotement (17.1, 17.2) du dispositif d'articulation de bras de support de telle sorte que l'axe d'appui est disposé spatialement de manière indépendante de la position de l'axe de pivotement au-dessus de l'axe de pivotement, de préférence décalé latéralement par rapport à l'axe de pivotement.
